# EUROPEAN PATENT SPECIFICATION

(11) **EP 4 260 276 B1**
(45) Date of publication and mention of the grant of the patent: **25.03.2026**
(21) Application number: 21823232.0
(22) Date of filing: 28.11.2021
(51) Int. Cl.: G06T 5/50, G06T 7/11, G06T 7/136, G06T 7/174, A61B 8/08

(54) **APPARATUS AND METHOD FOR CONTRAST IMAGING**
VORRICHTUNG UND VERFAHREN ZUR KONTRASTBILDGEBUNG
APPAREIL ET PROCÉDÉ D'IMAGERIE DE CONTRASTE

(30) Priority: 08.12.2020 US 202063122704 P
(43) Date of publication of application: 18.10.2023
(73) Proprietor: Koninklijke Philips N.V., 5656 AG Eindhoven (NL)
(72) Inventor: SHI, William, Tao, 5656 AE Eindhoven (NL); WANG, Shiying, 5656 AE Eindhoven (NL); ERRICO, Claudia, 5656 AE Eindhoven (NL); LOUPAS, Thanasis, 5656 AE Eindhoven (NL); TREMBLAY-DARVEAU, Charles, 5656 AE Eindhoven (NL); SHEERAN, Paul, 5656 AE Eindhoven (NL); POWERS, Jeffry, Earl, 5656 AE Eindhoven (NL)
(74) Representative: Philips Intellectual Property & Standards
(86) International application number: PCT/EP2021/083265
(87) International publication number: WO 2022/122429

(56) References cited:
- XIAOLIN GU ET AL: "Parametric perfusion imaging using contrast enhanced ultrasound with bolus administration of contrast agents", BIOMEDICAL AND HEALTH INFORMATICS (BHI), 2012 IEEE-EMBS INTERNATIONAL CONFERENCE ON, IEEE, 5 January 2012 (2012-01-05), pages 663 - 666, XP032449156, ISBN: 978-1-4577-2176-2, DOI: 10.1109/BHI.2012.6211670
- LOWERISON MATTHEW R ET AL: "A compound speckle model for vascular complexity quantification in nonlinear contrast-enhanced ultrasonography", 2014 IEEE INTERNATIONAL ULTRASONICS SYMPOSIUM, IEEE, 3 September 2014 (2014-09-03), pages 2249 - 2252, XP032667205, DOI: 10.1109/ULTSYM.2014.0560
- KAZUSHI NUMATA ET AL: "Use of accumulation images obtained by arterial-phase contrast-enhanced harmonic grayscale ultrasonography to evaluate tumor vessels in focal nodular hyperplasia and other hepatic tumors", JOURNAL OF MEDICAL ULTRASONICS, SPRINGER-VERLAG, TO, vol. 34, no. 1, 15 March 2007 (2007-03-15), pages 3 - 10, XP019476920, ISSN: 1613-2254, DOI: 10.1007/S10396-006-0132-9
- TANAKA HIRONORI: "Current role of ultrasound in the diagnosis of hepatocellular carcinoma", JOURNAL OF MEDICAL ULTRASONICS, JAPAN SOCIETY OF ULTRASONICS IN MEDICINE, TOKYO, JP, vol. 47, no. 2, 13 March 2020 (2020-03-13), pages 239 - 255, XP037105030, ISSN: 1346-4523, [retrieved on 20200313], DOI: 10.1007/S10396-020-01012-Y

## Description

### TECHNICAL FIELD

This application relates to contrast enhanced imaging. More specifically, this application relates to generating contrast accumulation images.

### BACKGROUND

Contrast-enhanced ultrasound (CEUS) has been utilized clinically for imaging organ and tumor vascularity as well as assessing tissue perfusion. A contrast agent is provided to an area or volume to be imaged in order to provide a higher signal strength from the area or volume of interest, or selectively enhance signals from areas or volumes with high contrast concentration. For example, microbubbles may be provided to a subject intravenously, and an area of interest, such as the liver, may be imaged by an ultrasound imaging system. The arrival, accumulation, and/or washout of the microbubbles in the area of interest may captured in ultrasound images acquired by the ultrasound imaging system.

Contrast accumulation imaging is a CEUS technique where multiple contrast-enhanced images (e.g., multiple image frames) are acquired in a temporal sequence, preprocessed by some number of image processing techniques, and then combined by some statistical operator (typically maximum value of a pixel over all frames) to form a single image (e.g., an accumulation image). The resulting image may be used to map contrast agent progression and/or enhance vessel topology and conspicuity.

Xiaolin Gu et al. (Proceedings of the IEEE-EMBS International Conference on Biomedical and Health Informatics 2012) discloses "Parametric perfusion imaging using contrast enhanced ultrasound with bolus administration of contrast agents".

Matthew R. Lowerison et al. (IEEE International Ultrasonics Symposium Proceedings, 2014) discloses "Use of accumulation images obtained by arterial-phase contrast-enhanced harmonic grayscale ultrasonography to evaluate tumor vessels in focal nodular hyperplasia and other hepatic tumors".

### SUMMARY

An apparatus and method for automatic frame selection for generating accumulation images are disclosed herein. As tissue signals are often suppressed incompletely on contrast-enhanced images, it is hard to differentiate the contrast echoes from tissue signals on grayscale contrast images. Consequently, image features within small moving windows on each of contrast images may be used for the automatic frame selection, instead of grayscale intensities of individual pixels. Image features are extracted from grayscale envelope statistical distributions within multiple-pixel windows across each contrast image frame. Examples of statistical distributions include, but are not limited to: Signal-to-noise ratio (SNR) as an exemplary first-order feature, a Nakagami Index (NI) as an exemplary second-order feature, and the product of the SNR and NI (SNR×NI) as an exemplary third-order feature. The moving window, and the grayscale envelope statistical distributions therein may be used to predict time of arrival of contrast (e.g., the first image frame), and the contrast enhancement will increase over time in intensity. The frame with peak intensity is as the last image frame. The apparatus and method disclosed herein may provide faster and more consistent frame selection for generating contrast accumulation images.

In some applications, the apparatuses, systems and methods for analyzing statistical distributions of pixels in images disclosed herein may be used for segmenting images. For example, segmenting tumors from images.

An apparatus for processing ultrasound images is defined by claim 1.

A method is defined by claim 8.

### BRIEF DESCRIPTION OF THE DRAWINGS

FIG. 1A is an example microvascular imaging (MVI) accumulation image.
FIG. 1B is an example of a time-of-arrival (ToA) accumulation image.
FIG. 2 is a block diagram of an ultrasound imaging system arranged in accordance with some examples of the present disclosure.
FIG. 3 is a block diagram illustrating an example processor in accordance with some examples of the present disclosure.
FIG. 4 illustrates analysis of image frames according to principles of the present disclosure.
FIG. 5 illustrates selection of image frames from a sequence of image frames according to principles of the present disclosure.
FIGS. 6A-9B are examples of contrast enhanced ultrasound image frames and parametric maps generated therefrom according to principles of the present disclosure.
FIG. 10 is a flow chart of a method according to principles of the present disclosure.

### DESCRIPTION

The following description of certain examples is merely exemplary in nature and is in no way intended to limit the invention or its applications or uses. In the following detailed description of examples of the present apparatuses, systems and methods, reference is made to the accompanying drawings which form a part hereof, and in which are shown by way of illustration specific examples in which the described apparatuses, systems and methods may be practiced. These examples are described in sufficient detail to enable those skilled in the art to practice the presently disclosed apparatuses, systems and methods, and it is to be understood that other examples may be utilized and that structural and logical changes may be made without departing from the scope defined by the appended claims. Moreover, for the purpose of clarity, detailed descriptions of certain features will not be discussed when they would be apparent to those with skill in the art so as not to obscure the description of the present system. The following detailed description is therefore not to be taken in a limiting sense, and the scope of the present system is defined only by the appended claims.

For accumulation imaging, a user may begin acquiring images with an ultrasound imaging system of a region of interest (ROI) in a subject and then administer a contrast agent, such as microbubbles. The contrast agent may be administered as a bolus or as a continuous infusion over a period of time. The user may continue to acquire images at the ROI for a period of time after administration. The imaging system may store the acquired images as a time-sequence. After the user has completed acquiring images, the user may select image frames from the stored time-sequence to combine to generate one or more accumulation images.

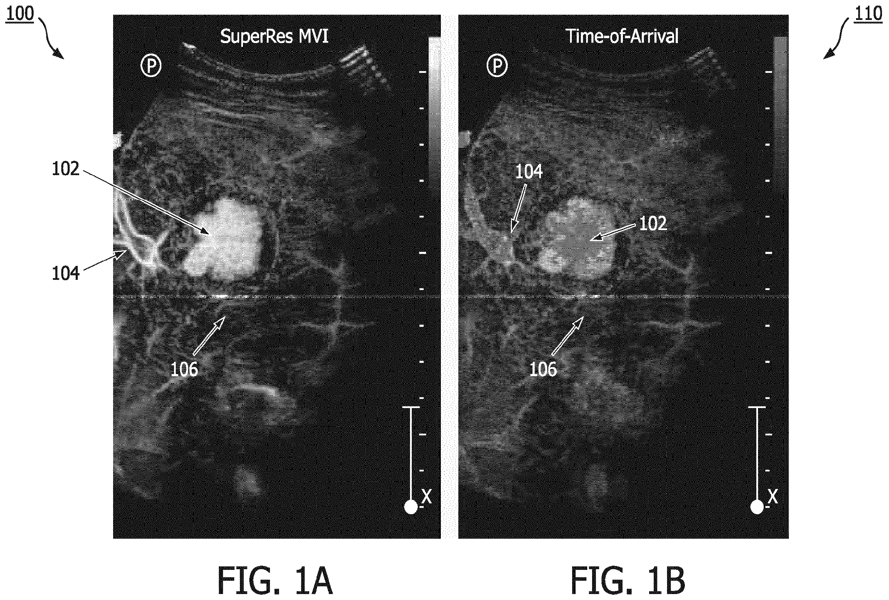

There are multiple techniques for accumulation imaging, which may provide different diagnostic information. For example, contrast accumulation imaging such as microvascular imaging (MVI) or maximum intensity projection (MIP) may combine multiple image frames to enhance visualization of smaller vasculature in a region of interest (ROI). An example of a MVI image is shown in FIG. 1A. Image 100 is a portion of a liver including a vascularized hepatic lesion (tumor) 102. Multiple ultrasound images were combined using MVI to produce the image 100 such that both larger vessels 104 and smaller vessels in the tumor 102 and vasculature 106 surrounding the tumor 102 are visualized. Examples of suitable techniques for performing MVI may be found in U.S. Provisional Application 62/938,554 filed November 21, 2019.

Another example is referred to as Time-of-Arrival (ToA) accumulation imaging. In ToA, different colors, grayscale values, and/or other coding is used to indicate when the contrast agent arrived in a particular region of the image, based on when the contrast agent appeared in the region in the sequence of image frames used to generate the accumulation image. **FIG. 1B** shows an example of a ToA image. Image 110 shows the same portion of the liver and tumor 102. However, different vasculature in image 110 are coded in different shades. As shown, larger vessels 104 and central regions of the tumor 102 are coded in a first shade, indicating contrast agent arrived in those regions first. The perimeter of the tumor 102 and surrounding smaller vasculature 106 are coded in a second shade indicating the contrast agent arrived in those regions later on. Examples of suitable techniques for performing ToA imaging may be found in U.S. Provisional Application 62/929,519 filed November 1, 2019.

In some applications, selection of the initial frame and/or the final frame used to define the image frames combined to generate the accumulation image may be important for ensuring diagnostic value of the resulting accumulation image. For example, an initial frame of a sequence for generating an accumulation image for ToA imaging is a frame where a contrast agent first appears. If an image frame in the sequence that occurs before or after the contrast agent first appears is selected, that may alter the apparent relative arrival of contrast agents to different regions of the tissue. This variance in calculated ToA's may make it difficult to compare ToA accumulation images between subjects and/or between exams of a same subject.

In another example, in both ToA and MVI, incorrect selection of the final frame may result in poor visualization of microvasculature. For example, if the final frame is too early in the sequence, the contrast agent may not have had time to accumulate in smaller vessels, resulting in poor visualization of microvasculature. On the other hand, if the final frame is too late in the sequence, contrast agent may have accumulated in the ROI such that the image is saturated, again resulting in poor visualization of the vasculature.

Currently, the user must manually review the image frames of the acquired time-sequence and manually select the first and last frames of a sequence of frames from the time-sequence to combine into an accumulation image. Manual review and selection of frames is time consuming for the user, particularly when a high frame rate was used and/or image frames were acquired over a long period of time. Furthermore, manual determination of the first and last image frames may be prone to human error, leading to inconsistent selection. This in turn may reduce diagnostic value of the resulting accumulation images. Accordingly, an automatic technique that provides more consistent frame selection is desirable.

Automatic selection methods based on grayscale intensity distributions of the acquired image frames are inadequate. CEUS images suffer from artefacts caused by incomplete tissue suppression. Residual tissue signals may be originated from multiple reflections within intervening tissue layers, strong reflections from tissue interfaces (e.g., large vessel walls, abdominal membranes), and/or dissimilarity between transmit pulses used stimulate the contrast agent. Furthermore, residual tissue signals cannot generally be removed with simple frame subtraction because of tissue motion and acoustic signal incoherence.

The present disclosure is directed to apparatuses, systems, and methods for extracting image features from grayscale envelope statistical distributions within multi-pixel windows across each contrast image frame. That is, statistical features of groups of pixels are analyzed rather than grayscale values (e.g., intensities) of individual pixels for feature extraction. Extraction of features from the statistical distributions may permit analysis of contrast image frames for selection of first and/or last frames of a sequence for generating an accumulation image. The grayscale envelope statistical distributions may be used to select a first frame in a sequence where the contrast agent appears (e.g., time of arrival). As contrast enhancement is increased over time, the contrast will peak in intensity in one of the image frames, which may also be detected by the grayscale envelope statistical distributions therein. The frame with peak intensity (e.g., the first frame where peak intensity is reached) is used as the last image frame of the sequence. In some examples, the frame with the peak intensity may be used to determine a frame at a midpoint (time-wise) between the frame with the peak intensity and the first frame, and the frame at the midpoint may be selected as the last frame. Other techniques for selecting the last frame may also be used (e.g., predetermined number of frames after the first frame, finding frame with ½ or ¼ of peak intensity). Which technique is used may be based, at least in part, on the anatomy being imaged (e.g., liver, breast, or heart) and/or type of accumulation imaging (e.g., MVI or ToA). The apparatuses, systems, and methods disclosed herein may provide faster and/or more consistent frame selection for generating contrast accumulation images.

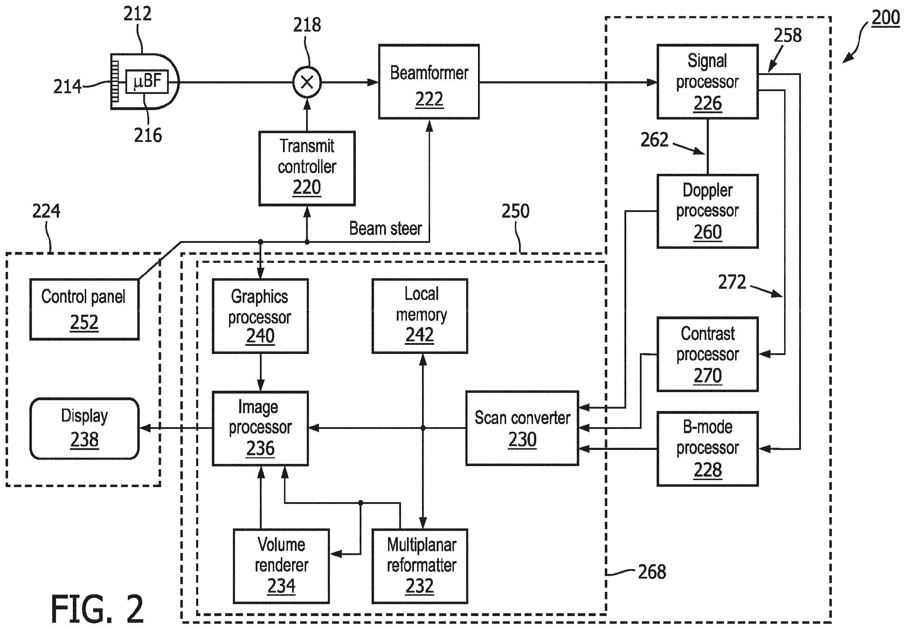

**FIG. 2** shows a block diagram of an ultrasound imaging system 200 constructed in accordance with the principles of the present disclosure. An ultrasound imaging system 200 according to the present disclosure may include a transducer array 214, which may be included in an ultrasound probe 212, for example an external probe or an internal probe. The transducer array 214 is configured to transmit ultrasound signals (e.g., beams, waves) and receive echoes (e.g., received ultrasound signals) responsive to the transmitted ultrasound signals. A variety of transducer arrays may be used, e.g., linear arrays, curved arrays, or phased arrays. The transducer array 214, for example, can include a two dimensional array (as shown) of transducer elements capable of scanning in both elevation and azimuth dimensions for 2D and/or 3D imaging. As is generally known, the axial direction is the direction normal to the face of the array (in the case of a curved array the axial directions fan out), the azimuthal direction is defined generally by the longitudinal dimension of the array, and the elevation direction is transverse to the azimuthal direction.

In some examples, the transducer array 214 may be coupled to a microbeamformer 216, which may be located in the ultrasound probe 212, and which may control the transmission and reception of signals by the transducer elements in the array 214. In some examples, the microbeamformer 216 may control the transmission and reception of signals by active elements in the array 214 (e.g., an active subset of elements of the array that define the active aperture at any given time).

In some examples, the microbeamformer 216 may be coupled, e.g., by a probe cable or wirelessly, to a transmit/receive (T/R) switch 218, which switches between transmission and reception and protects the main beamformer 222 from high energy transmit signals. In some examples, for example in portable ultrasound systems, the T/R switch 218 and other elements in the system can be included in the ultrasound probe 212 rather than in the ultrasound system base, which may house the image processing electronics. An ultrasound system base typically includes software and hardware components including circuitry for signal processing and image data generation as well as executable instructions for providing a user interface.

The transmission of ultrasonic signals from the transducer array 214 under control of the microbeamformer 216 is directed by the transmit controller 220, which may be coupled to the T/R switch 218 and a main beamformer 222. The transmit controller 220 may control the direction in which beams are steered. Beams may be steered straight ahead from (orthogonal to) the transducer array 214, or at different angles for a wider field of view. The transmit controller 220 may also be coupled to a user interface 224 and receive input from the user's operation of a user control. The user interface 224 may include one or more input devices such as a control panel 252, which may include one or more mechanical controls (e.g., buttons, encoders, etc.), touch sensitive controls (e.g., a trackpad, a touchscreen, or the like), and/or other known input devices.

In some examples, the partially beamformed signals produced by the microbeamformer 216 may be coupled to a main beamformer 222 where partially beamformed signals from individual patches of transducer elements may be combined into a fully beamformed signal. In some examples, microbeamformer 216 is omitted, and the transducer array 214 is under the control of the beamformer 222 and beamformer 222 performs all beamforming of signals. In examples with and without the microbeamformer 216, the beamformed signals of beamformer 222 are coupled to processing circuitry 250, which may include one or more processors (e.g., a signal processor 226, a B-mode processor 228, a Doppler processor 260, and one or more image generation and processing components 268) configured to produce an ultrasound image from the beamformed signals (i.e., beamformed RF data).

The signal processor 226 may be configured to process the received beamformed RF data in various ways, such as bandpass filtering, decimation, I and Q component separation, and harmonic signal separation. The signal processor 226 may also perform additional signal enhancement such as speckle reduction, signal compounding, and electronic noise elimination. The processed signals (also referred to as I and Q components or IQ signals) may be coupled to additional downstream signal processing circuits for image generation. The IQ signals may be coupled to a plurality of signal paths within the system, each of which may be associated with a specific arrangement of signal processing components suitable for generating different types of image data (e.g., B-mode image data, contrast image data, Doppler image data).

For example, the system 200 may include a B-mode signal path 258 which couples the signals from the signal processor 226 to a B-mode processor 228 for producing B-mode image data for contrast and/or regular grayscale images. The B-mode processor 228 can employ amplitude detection for the imaging of organ structures the body. In another example, the system 200 may include a contrast signal path 272 which couples the signals from the signal processor 226 to a contrast processor 270 for producing contrast image data. The contrast processor 270 may employ amplitude detection, harmonic imaging techniques, and/or other processing techniques for detection of contrast agent (e.g., microbubbles) in the body. In some examples, the B-mode processor 228 and contrast processor 270 may be implemented by a single processor.

The signals produced by the B-mode processor 228 and/or the contrast processor 270 may be coupled to a scan converter 230 and/or a multiplanar reformatter 232. The scan converter 230 may be configured to arrange the echo signals from the spatial relationship in which they were received to a desired image format. For instance, the scan converter 230 may arrange the echo signal into a two dimensional (2D) sector-shaped format, or a pyramidal or otherwise shaped three dimensional (3D) format. The multiplanar reformatter 232 can convert echoes which are received from points in a common plane in a volumetric region of the body into an ultrasonic image (e.g., a B-mode image) of that plane, for example as described in U.S. Pat. No. 6,443,896 (Detmer). The scan converter 230 and multiplanar reformatter 232 may be implemented as one or more processors in some examples.

A volume renderer 234 may generate an image (also referred to as a projection, render, or rendering) of the 3D dataset as viewed from a given reference point, e.g., as described in U.S. Pat. No. 6,530,885 (Entrekin et al.). The volume renderer 234 may be implemented as one or more processors in some examples. The volume renderer 234 may generate a render, such as a positive render or a negative render, by any known or future known technique such as surface rendering and maximum intensity rendering. Although shown in FIG. 2 as receiving data from the multiplanar reformatter 232, in some examples, the volume renderer 234 may receive data from the scan converter 230.

In some examples, the system may include a Doppler signal path 262 which couples the output from the signal processor 226 to a Doppler processor 260. The Doppler processor 260 may be configured to estimate the Doppler shift and generate Doppler image data. The Doppler image data may include color data which is then overlaid with B-mode (i.e. grayscale) image data for display. The Doppler processor 260 may be configured to filter out unwanted signals (i.e., noise or clutter associated with non-moving tissue), for example using a wall filter. The Doppler processor 260 may be further configured to estimate velocity and power in accordance with known techniques. For example, the Doppler processor may include a Doppler estimator such as an auto-correlator, in which velocity (Doppler frequency) estimation is based on the argument of the lag-one autocorrelation function and Doppler power estimation is based on the magnitude of the lag-zero autocorrelation function. Motion can also be estimated by known phase-domain (for example, parametric frequency estimators such as MUSIC, ESPRIT, etc.) or time-domain (for example, cross-correlation) signal processing techniques. Other estimators related to the temporal or spatial distributions of velocity such as estimators of acceleration or temporal and/or spatial velocity derivatives can be used instead of or in addition to velocity estimators. In some examples, the velocity and power estimates may undergo further threshold detection to further reduce noise, as well as segmentation and post-processing such as filling and smoothing. The velocity and power estimates may then be mapped to a desired range of display colors in accordance with a color map. The color data, also referred to as Doppler image data, may then be coupled to the scan converter 230, where the Doppler image data may be converted to the desired image format and overlaid on the B-mode image of the tissue structure to form a color Doppler or a power Doppler image. For example, Doppler image data may be overlaid on a B-mode image of the tissue structure.

Output (e.g., B-mode images, contrast images, Doppler images) from the scan converter 230, the multiplanar reformatter 232, and/or the volume renderer 234 may be coupled to an image processor 236 for further enhancement, buffering and temporary storage before being displayed on an image display 238.

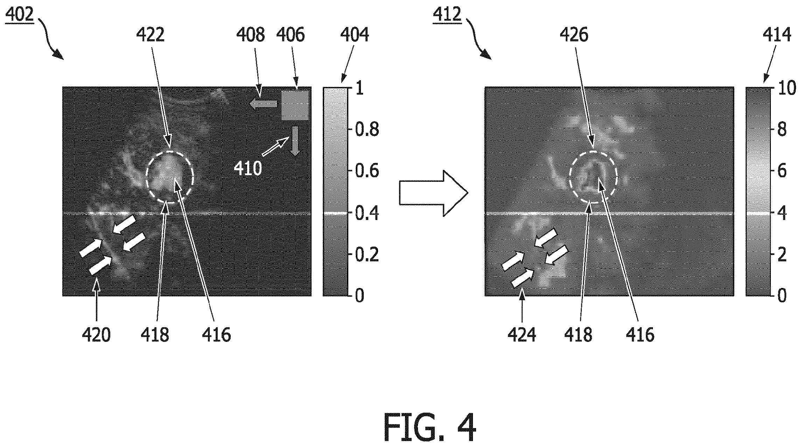

The image processor 236 receives imaging data corresponding to image frames of a sequence (e.g., multi-frame loop, cineloop) of contrast enhanced images. Each image frame in the sequence has been acquired at a different time (e.g., the image frames may be temporally spaced). The image processor 236 combines two or more of the image frames in the sequence to generate an accumulation image. The image processor 236 analyzes each image frame in the sequence to select an image frame from the sequence and a later image frame from the sequence. The image frame and the later image frame are first and last frames, respectively, of a subset of image frames from the sequence (e.g., subsequence). The first image frame, the last image frame, and the images acquired between the first and last image frames (e.g., the frames of the subsequence) are combined to generate the accumulation image. In some examples, the image processor 236 may analyze each frame in the sequence to segment one or more features from the images. For example, a tumor or other features of interest may be segmented. Analysis of the image frames is described in more detail with reference to FIGS. 4-10. The accumulation image and/or results of analysis of the image frames may be provided to the display 238 and/or local memory 242.

A graphics processor 240 may generate graphic overlays for display with images, such as the accumulation image generated by the image processor 236. These graphic overlays can contain, e.g., standard identifying information such as patient name, date and time of the image, imaging parameters, and the like. For these purposes the graphics processor may be configured to receive input from the user interface 224, such as a typed patient name or other annotations. The user interface 224 can also be coupled to the multiplanar reformatter 232 for selection and control of a display of multiple multiplanar reformatted (MPR) images.

The system 200 may include local memory 242. Local memory 242 may be implemented as any suitable non-transitory computer readable medium (e.g., flash drive, disk drive). Local memory 242 may store data generated by the system 200 including B-mode images, parametric maps, executable instructions, inputs provided by a user via the user interface 224, or any other information necessary for the operation of the system 200.

As mentioned previously, system 200 includes user interface 224. User interface 224 may include display 238 and control panel 252. The display 238 may include a display device implemented using a variety of known display technologies, such as LCD, LED, OLED, or plasma display technology. In some examples, display 238 may comprise multiple displays. The control panel 252 may be configured to receive user inputs (e.g., exam type, time of contrast agent injection, selection of ROI in image). The control panel 252 may include one or more hard controls (e.g., buttons, knobs, dials, encoders, mouse, trackball or others). In some examples, the control panel 252 may additionally or alternatively include soft controls (e.g., GUI control elements or simply, GUI controls) provided on a touch sensitive display. In some examples, display 238 may be a touch sensitive display that includes one or more soft controls of the control panel 252.

In some examples, various components shown in FIG. 2 may be combined. For instance, image processor 236 and graphics processor 240 may be implemented as a single processor. In another example, the scan converter 230 and multiplanar reformatter 232 may be implemented as a single processor. In some examples, various components shown in FIG. 2 may be implemented as separate components. For example, signal processor 226 may be implemented as separate signal processors for each imaging mode (e.g., B-mode, Contrast, Doppler). In some examples, one or more of the various processors shown in FIG. 2 may be implemented by general purpose processors and/or microprocessors configured to perform the specified tasks. In some examples, one or more of the various processors may be implemented as application specific circuits. In some examples, one or more of the various processors (e.g., image processor 236) may be implemented with one or more graphical processing units (GPU).

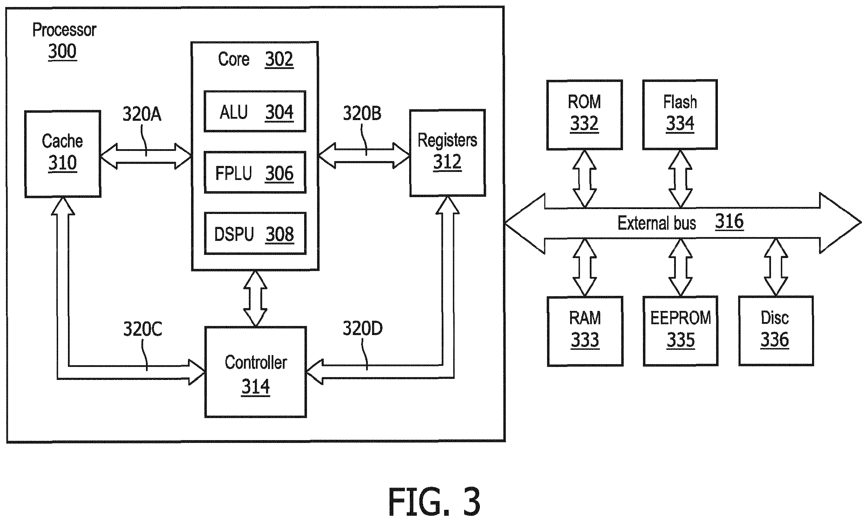

**FIG. 3** is a block diagram illustrating an example processor 300 according to principles of the present disclosure. Processor 300 may be used to implement one or more processors described herein, for example, image processor 236 shown in FIG. 1. Processor 300 may be any suitable processor type including, but not limited to, a microprocessor, a microcontroller, a digital signal processor (DSP), a field programmable array (FPGA) where the FPGA has been programmed to form a processor, a graphical processing unit (GPU), an application specific circuit (ASIC) where the ASIC has been designed to form a processor, or a combination thereof.

The processor 300 may include one or more cores 302. The core 302 may include one or more arithmetic logic units (ALU) 304. In some examples, the core 302 may include a floating point logic unit (FPLU) 306 and/or a digital signal processing unit (DSPU) 308 in addition to or instead of the ALU 304.

The processor 300 may include one or more registers 312 communicatively coupled to the core 302. The registers 312 may be implemented using dedicated logic gate circuits (e.g., flip-flops) and/or any memory technology. In some examples the registers 312 may be implemented using static memory. The register may provide data, instructions and addresses to the core 302.

In some examples, processor 300 may include one or more levels of cache memory 310 communicatively coupled to the core 302. The cache memory 310 may provide computer-readable instructions to the core 302 for execution. The cache memory 310 may provide data for processing by the core 302. In some examples, the computer-readable instructions may have been provided to the cache memory 310 by a local memory, for example, local memory attached to the external bus 316. The cache memory 310 may be implemented with any suitable cache memory type, for example, metal-oxide semiconductor (MOS) memory such as static random access memory (SRAM), dynamic random access memory (DRAM), and/or any other suitable memory technology.

The processor 300 may include a controller 314, which may control input to the processor 300 from other processors and/or components included in a system (e.g., control panel 252 and scan converter 230 shown in FIG. 1) and/or outputs from the processor 300 to other processors and/or components included in the system (e.g., display 238 and volume renderer 234 shown in FIG. 1). Controller 314 may control the data paths in the ALU 304, FPLU 306 and/or DSPU 308. Controller 314 may be implemented as one or more state machines, data paths and/or dedicated control logic. The gates of controller 314 may be implemented as standalone gates, FPGA, ASIC or any other suitable technology.

The registers 312 and the cache 310 may communicate with controller 314 and core 302 via internal connections 320A, 320B, 320C and 320D. Internal connections may implemented as a bus, multiplexor, crossbar switch, and/or any other suitable connection technology.

Inputs and outputs for the processor 300 may be provided via a bus 316, which may include one or more conductive lines. The bus 316 may be communicatively coupled to one or more components of processor 300, for example the controller 314, cache 310, and/or register 312. The bus 316 may be coupled to one or more components of the system, such as display 238 and control panel 252 mentioned previously.

The bus 316 may be coupled to one or more external memories. The external memories may include Read Only Memory (ROM) 332. ROM 332 may be a masked ROM, Electronically Programmable Read Only Memory (EPROM) or any other suitable technology. The external memory may include Random Access Memory (RAM) 333. RAM 333 may be a static RAM, battery backed up static RAM, Dynamic RAM (DRAM) or any other suitable technology. The external memory may include Electrically Erasable Programmable Read Only Memory (EEPROM) 335. The external memory may include Flash memory 334. The external memory may include a magnetic storage device such as disc 336. In some examples, the external memories may be included in a system, such as ultrasound imaging system 200 shown in FIG. 2, for example local memory 242.

In some examples, processor 300 may be included in a computing system separate from an ultrasound imaging system, such as system 200. For example, the computing system may be a workstation for post-ultrasound exam processing of images acquired during the exam. In these examples, the processor 300 may perform some or all of the processing described as being performed by image processor 236 in system 200. For example, the analysis and selection of image frames, generating accumulation images, and/or segmenting features from the image frames.

FIG. 4 illustrates analysis of image frames according to principles of the present disclosure. In some examples, the analysis may be performed by a processor, such as image processor 236 shown in FIG. 2. Rather than analyzing a value (e.g., grayscale value, intensity value) for each individual pixel of an image frame 402, statistical distributions of values of groups of pixels are analyzed. As shown in FIG. 4, a moving window 406 is translated across the image 402 as indicated by arrows 408 and 410. The moving window 406 may include any number of pixels, for example, the moving window 406 may have dimensions of 20x20 pixels (total of 400 pixels) or 16x16 pixels (total of 256 pixels). The window 406 may not be square in some examples (e.g., circular, rectangular). The size and/or shape of the window 406 may be based, at least in part, on a size of the image 402. For example, a 16x16 pixel moving window may be selected for a 600x800 pixel image 402.

Each time the window 406 is translated across the image 402, a statistical distribution of the values of the pixels is analyzed and a parametric value is generated. The parametric value is then assigned to a center of the window 406 (e.g., having a location equivalent to a pixel at the center of the window 406). Various parameters and/or combinations thereof may be calculated for the parametric value. For example, the signal-to-noise ratio (SNR) may be calculated for each window 406. In another example, the Nakagami Index (NI) may be calculated for each window 406. In a further example, both the SNR and the NI may be calculated and the final parametric value may be a product of the SNR and NI. These parameters are merely exemplary and other distributions or combinations thereof may be used (e.g., gamma, Hoyt) in other examples. The parametric values may be used to generate a parametric map 412. In some examples, a number of parametric values calculated may be equal to a number of pixels in the image 402. That is, each pixel of image 402 may have a corresponding parametric value. Thus, the parametric map 412 may have the same dimensions as the image 402 in some examples. In the example shown in FIG. 4, the parametric map 412 illustrates the SNR for image 402. A parametric map, such as parametric map 412 may be generated for each frame in a sequence of frames.

Optionally, in some examples, rather than translating window 406 across the entire image 402, a large ROI (not shown) may be selected in image 402 either automatically and/or by a user via a user input through a user interface, such as user interface 224. In these examples, the window 406 may be translated through the ROI rather than the entire image 402, and parametric map 412 may only be generated for the ROI. This may be advantageous in applications where significant artefacts are present in the image 402. The ROI may be chosen to exclude regions including the artefacts (e.g., distortions from a nearby implanted device, skin surface reflections).

Optionally, in some examples, an image frame that is known to be acquired prior to contrast agent arrival (e.g., prior to contrast agent injection, at least two seconds before expected contrast agent arrival) may be used as a mask applied to image 402 which may exclude some or all areas in image 402 including residual tissue signals from the parametric map 412. In some examples, the user may provide an input via the user interface to indicate when contrast agent is injected, which may be used to determine which image frame to use as a mask.

By generating parametric maps 412, the dynamic range of values associated with each image frame 402 may be increased. In the example shown, for a limited dynamic range from 0-1 (as shown by the scale bar 404) for the original grayscale image 402, the grayscale values for residual tissue signals from the abdominal membrane (indicated by arrows 420) are comparable to the grayscale values for strong contrast echoes inside the tumor (indicated by the dashed circle 422). For an enlarged dynamic range from 0-10 (as shown by the scale bar 414), an order of magnitude greater, for the parametric map 412, the parametric values for the residual tissue signals from the abdominal membrane (indicated by arrows 424) are hardly visible and much lower than the parametric values for the strong contrast echoes inside the tumor (indicated by the dashed circle 426). By increasing the dynamic range, it may be easier to distinguish between signals from contrast agent and signals from tissue. For example, there is a greater difference between pixel values for tumor 416 within ROI 418 in image 402 than in image 402. By increasing the difference between values for tissue and contrast agent, it may be easier to automatically select first and last frames from a sequence to form a subsequence to generate an accumulation image.

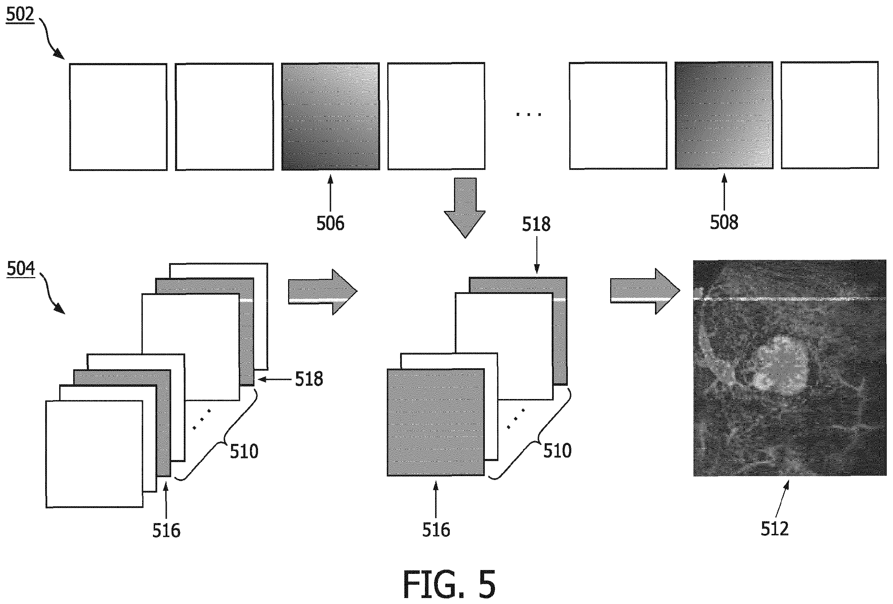

**FIG. 5** illustrates selection of image frames from a sequence of image frames according to principles of the present disclosure. In some examples, the selection may be performed by a processor, such as image processor 236 shown in FIG. 2. A sequence of parametric maps 502 corresponds to a sequence of image frames 504. The parametric maps of the sequence 502 are analyzed to determine whether any of the parametric maps has a value equal to or above a threshold value. The threshold value may be selected based, at least in part, on a maximum value for the parametric for the tissue type(s) being imaged. For example, in liver imaging, when SNR or NI is used as the parameter, the threshold value may be equal to 4, and when SNRxNI is used as the parameter, the threshold value may be equal to 16. Other values may be selected for the threshold value when different tissue is imaged (e.g., breast tissue, heart tissue) and/or different parameters are used. In some examples, a user may set the threshold value. In some examples, the threshold value may be based on an exam type and/or an indication by the user what type(s) of tissue is included in the image frames.

The earliest acquired parametric map to have a value equal to or greater than the threshold value, parametric map 506 in the example shown, is used to select a first image frame 516 of a subsequence 510 from the image sequence 504. In some examples, image frame 516 may be an image frame corresponding to the parametric map 506. That is, parametric map 506 was generated from image frame 516 as described with reference to FIG. 4. In some examples, image frame 516 may be an image frame acquired at a predetermined time or a predetermined number of frames prior to (or after) the image frame used to generate parametric map 506. For example, image frame 516 may be an image frame acquired one second before (or after) the image frame used to generate parametric map 506. In another example, image frame 516 may be an image frame acquired 100 frames before (or after) the image frame used to generate parametric map 506.

The sequence of parametric maps 502 is further analyzed to determine which frame(s) include a maximum (e.g., peak) parametric value of the parametric values included in the sequence of parametric maps 502. In some examples, the earliest acquired parametric map including the maximum parametric value, parametric map 508 in the example shown, may be used to select a last frame 518 of the subsequence 510. In some examples, image frame 518 may be an image frame corresponding to the parametric map 508. In some examples, the maximum parametric value found in parametric map 508 may be used to calculate a parametric value equal to some fraction of the maximum value (e.g., ½, ¼). The parametric maps of the sequence 502 may then be analyzed to find the earliest acquired parametric map including the calculated parametric value, and the corresponding image frame may be selected as image frame 518.

Alternatively, instead of analyzing the sequence of parametric maps 502 to find a maximum parametric value, the image frame 518 may be selected based, at least in part, on image frame 516. For example, an image frame acquired after a predetermined amount of time after image frame 516 may be selected as image frame 518. In another example, an image frame acquired at predetermined number of frames after image frame 516 may be selected as image frame 518.

Once image frame 516 and image frame 518 have been selected, the image frames 516, image frame 518, and image frames acquired between image frames 516 and 518, are included in subsequence 510. The image frames of subsequence 510 are combined to generate an accumulation image 512. In some examples, such as the one shown in FIG. 5, accumulation image 512 may be a ToA accumulation image where contrast agent is coded (e.g., different intensities or colors) to indicate which regions the contrast agent arrived at earlier or later relative to other regions. However, other types of accumulation images may be generated from subsequence 510 in other examples.

Optionally, in some examples, a user may view the automatically selected frames 516 and 518 and override one or both selections. The user may then select a different image frame from sequence 504 for image frame 516 and/or image frame 518. Allowing the user to override the selection may be advantageous in some situations, for example, when an incorrect threshold value or tissue type was provided by the user.

Although FIG. 5 describes analyzing a single sequence of parametric maps 502, in some examples, multiple sequences may be analyzed. For example, parametric maps may be generated based on multiple parameters (e.g., SNR, NI).

The techniques disclosed herein for automatically selecting the first and final image frames of a sequence of image frames for generating an accumulation image may reduce or eliminate the need for manual review of image frames of a sequence to select images for generating an accumulation image. The techniques disclosed herein may provide for more consistent selection of first and last images of the sequence used to generate the accumulation image. In some applications, this may improve the consistency and/or diagnostic value of the accumulation image.

Although the parametric maps disclosed with reference to FIGS. 4 and 5 have been described for use in selecting the first and last frames for generating accumulation images, the parametric maps may be used for other applications in contrast imaging. For example, the range scale and rate of temporal change of the parametric values in the parameter maps may be used to control the range scale and temporal gradient of time-varying coding for ToA imaging. For example, the temporal gradient may be proportional to the temporal gradient of image features in the parametric maps. For fast changes in contrast images, a higher temporal gradient in image features may provide a higher temporal gradient in the coding (e.g., color range, grayscale intensity) with a larger coding range in a short time frame. For slow changes, a lower temporal gradient may lead to a lower temporal gradient in the coding with a smaller coding range in a longer time range. This adaptation of the coding for ToA may provide better visualization of fast and slow processes (e.g., rapid arrival and slow uptake in tissue), respectively.

In some examples, the parametric maps may be used for image segmentation (e.g., segmenting features from images). For example, as noted in reference to FIG. 4, the parametric values of tumor 416 have a much higher intensity compared to the surrounding tissue than the intensity values of pixels of tumor 416 compared to the intensity values of pixels of surrounding tissue. That is, the differences of values between normal tissue and tumor may be greater in the parametric maps than in the original image frames. Accordingly, certain segmentation techniques, such as those based, at least in part, on threshold values to distinguish between tissue types, may be more reliable when applied to the parametric maps rather than directly to the original image frames.

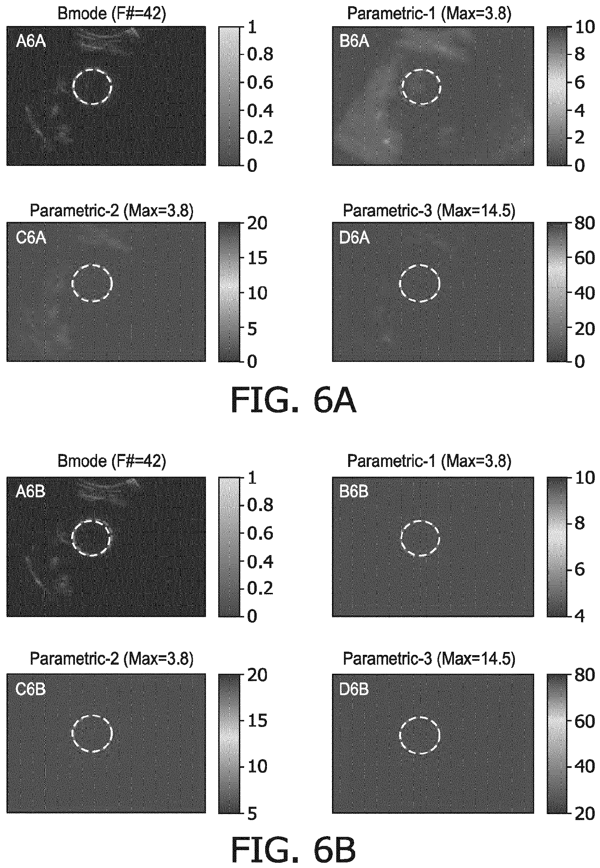

**FIGS. 6A-9B** are examples of contrast enhanced ultrasound image frames and parametric maps generated therefrom according to principles of the present disclosure. The ultrasound images and parametric maps shown in FIGS. 6A-9B include the same hepatic lesion (tumor) as shown in FIGS. 1A and 1B. The ultrasound images A6A-A6B, A7A-A7B, A8A-A8B, and A9A-A9B shown in Figures 6A-9B are example contrast enhanced images from a sequence of contrast enhanced images. In some examples, the ultrasound images may have been acquired by an ultrasound imaging system, such as system 200. The parametric maps may have been generated by a processor, such as image processor 236 in some examples.

**FIGS. 6A-6B** show a 42^{nd} image frame A6A-A6B from the sequence. The image frame A6A-A6B was acquired approximately one second before contrast agent arrived in the region included in the image frame. In FIG. 6A, parametric maps B6A, C6A, and D6A were generated from image frame A6A by a 16x16 moving pixel window and analyzing the statistical distribution to determine parametric values. Parametric map B6A shows values for SNR, parametric map C6A shows values for NI, and parametric map D6A shows values for SNRxNI. As can illustrated by the scale bars on the right hand side of the image frame A1 and the parametric maps B6A-D6A, all three parametric maps have a greater dynamic range than the image frame.

The parametric maps B6A-D6A in **FIG. 6A** include parametric values based on signals from the tissue and artefacts since the contrast agent has not yet arrived. The maximum signal in parametric maps B6A and C6A is 3.8 and the maximum value parametric map D6A is 14.5. Accordingly, since no contrast agent is present, these maximum values from the tissue and other artefacts may be used to set a threshold value. For example, the threshold value for parametric maps B6A and C6A may be 4, whereas the threshold value for parametric map D6A may be 16.

**FIG. 6B** illustrates the same image frame and parametric maps as shown in FIG. 6A. However, the parametric maps B6B-D6B have been thresholded to remove any values below the threshold values shown above. Thus, all three parametric maps in FIG. 6B have zero for all parametric values.

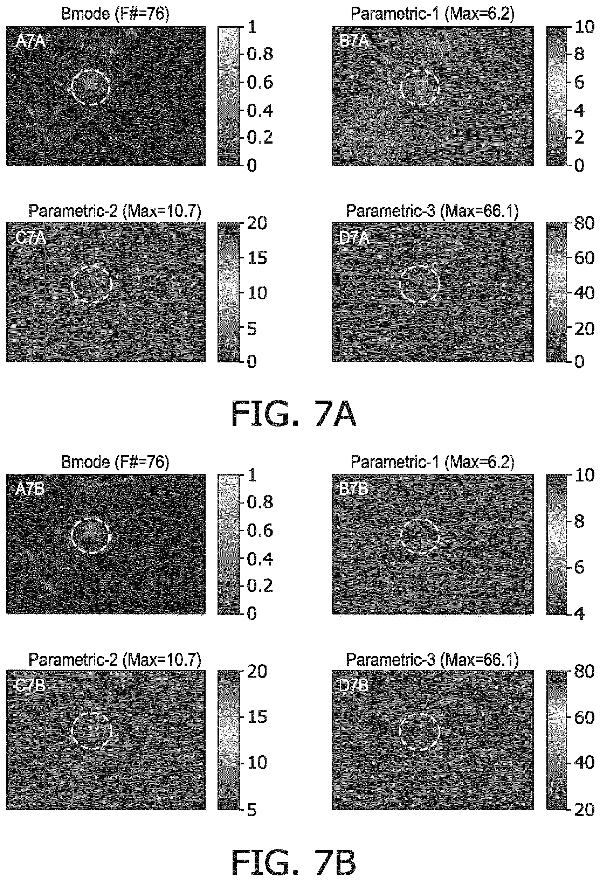

**FIGS. 7A-7B** show a 76^{th} image frame A7A-A7B from the sequence. The image frame A7A-A7B was acquired approximately one second after contrast agent arrived in the region included in the image frame. Parametric maps B7A-D7A in FIG. 7A were generated in a similar manner as described with reference to FIG. 6A. As can be seen in all three parametric maps, the maximum parametric values have increased. However, the parametric values at the tissue sites are comparable to those at the tumor site indicated by the dashed circle. FIG. 7B shows parametric maps B7B-D7B, which have been thresholded in a similar manner to the parametric maps B6B-D6B shown in FIG. 6B. The threshold values determined from the parametric maps B6A-D6A in FIG. 6A were used. After thresholding, only the signal from the contrast agent in the tumor is visible while all tissue signals are removed.

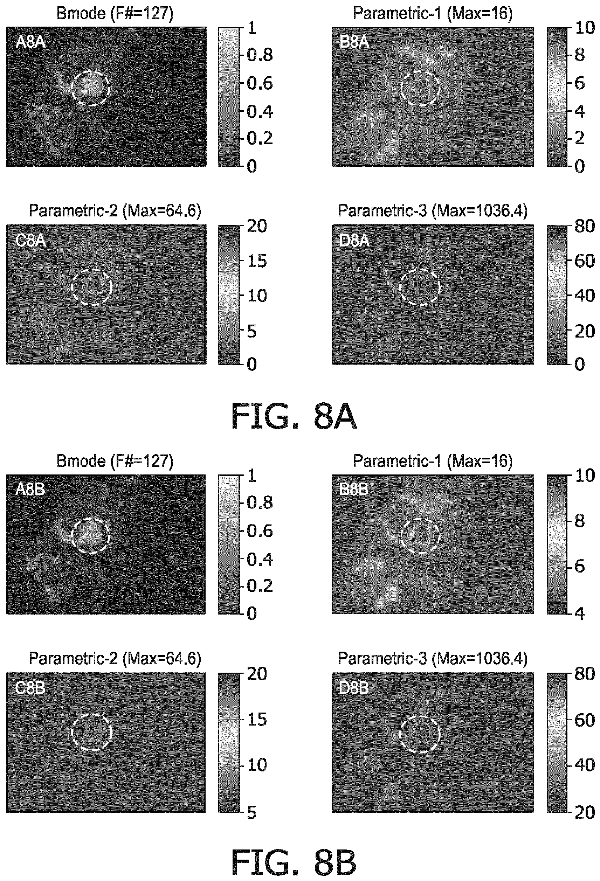

**FIGS. 8A-8B** show a 127^{th} image frame A8A-A8B from the sequence. The image frame A8A-A8B was acquired approximately four seconds after contrast agent arrived in the region included in the image frame. Parametric maps B8A-D8A in FIG. 8A were generated in a similar manner as described with reference to FIG. 6A. As can be seen in all three parametric maps, the maximum parametric values have now significantly increased, particularly at the tumor site in the dashed circle. The parametric values at the tumor site are now significantly greater than the parametric values at the tissue sites. As shown in FIG. 8B, when the parametric maps B8A-D8A are thresholded to generate parametric maps B8B-D8B, most of the tumor site remains visible in the parametric maps, and there is little to no tissue noise surrounding the tumor site.

The thresholded parametric maps B8B-D8B may be used for segmenting the tumor from the image frame in some examples. For example, pixels in image frame A8A-A8B corresponding to parametric values above the threshold value may be categorized as belonging to the tumor. In some examples, only pixels corresponding to parametric values above the threshold value within an ROI, such as the dashed circle, may be assigned to the tumor. Segmenting may be performed based on one or more of the parametric maps B8B-D8B.

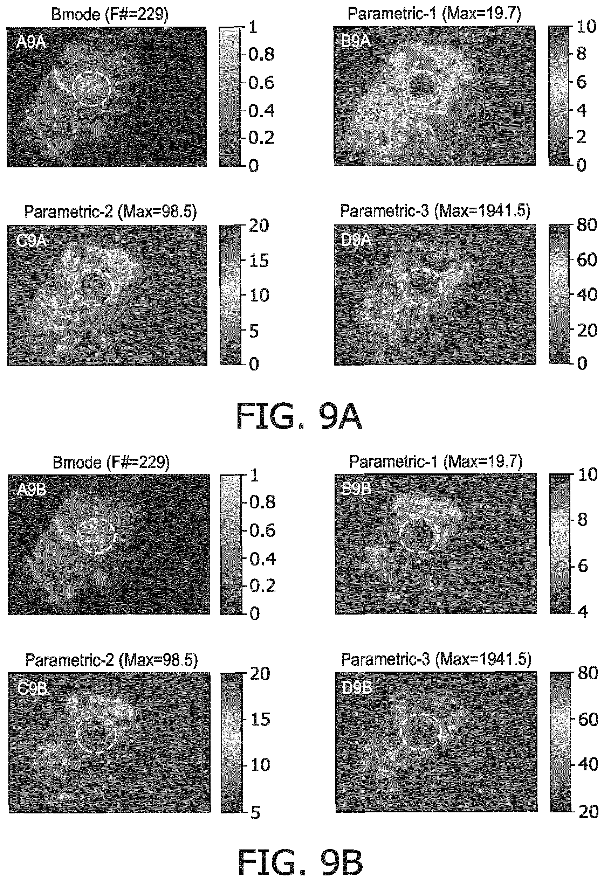

**FIGS. 9A-9B** show a 229^{th} image frame A9A-A9B from the sequence. The image frame A9A-A9B was acquired approximately ten seconds after contrast agent arrived in the region included in the image frame. Parametric maps B9A-D9A in FIG. 9A were generated in a similar manner as described with reference to FIG. 6A. As can be seen in all three parametric maps, the maximum parametric values have continued to increase, but now in the tissue surrounding the tumor site indicated by the dashed circle. This is due to contrast agent accumulating in the tissue. However, as shown in FIG. 9B, when the parametric maps are thresholded B9A-D9A to generate parametric maps B9A-D9B, clutter around the tumor is still reduced. Thus, segmenting the tumor from the image frame may still be improved by the parametric maps B9A-D9B.

In **FIGS. 9A-9B** the maximal parametric value (peak) can be found inside the tumor (within the dashed circle). General speaking, contrast kinetics (e.g., arrival time and peak time) are different at different locations. For an entire image with a complex contrast kinetic distribution, the initial appearance of contrast signals (grayscale intensity and parametric values) anywhere on the image can be considered as the arrival time and the maximal contrast echoes (grayscale intensity and parametric values) over the entire image as the peak time.

Accordingly, in addition to selecting first and last image frames of a sequence for generating an accumulation image, parametric maps may also or alternatively be used for segmenting tumors or other objects of interest from image frames.

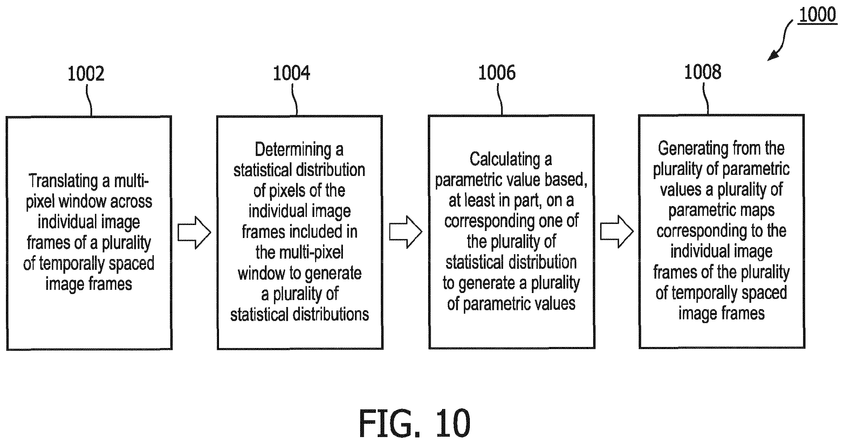

**FIG. 10** is a flow chart of a method according to principles of the present disclosure. The flow chart 1000 summarizes the analysis and selection techniques described herein, such as those described with reference to FIGS. 4-9B. In some examples, the method shown in flow chart 1000 may be performed by one or more components of an ultrasound imaging system, such as ultrasound imaging system 200. For example, an image processor, such as image processor 236 may perform some or all of the method shown in flow chart 1000. In some examples, the method shown in flow chart 1000 may be performed by a processor included in a computing system separate from ultrasound imaging system 200. The computing system may include a processor, such as processor 300, and/or a processor equivalent to processor 236 for post-processing of images acquired during an exam.

In some examples, at least one processor, such as image processor 236, may translate a multi-pixel window across individual image frames of a plurality of temporally spaced image frames as indicated at block 1002. The image frames are ultrasound image frames. The image frames are CEUS images.

For each translation of the multi-pixel window, as indicated by block 1004, the at least one processor determines a statistical distribution of pixels of the individual image frames included in the multi-pixel window to generate a plurality of statistical distributions. In some examples, the multi-pixel window may be a square. In some examples, at least one processor may translate the multi-pixel window one pixel at a time. In some examples, the at least one processor may translate the multi-pixel window a number of times such that the number of statistical distributions calculated for each image frame equals the number of pixels in the image frame. In some examples, the at least one processor may only translate the multi-pixel window across a ROI in the image frames. In some examples, the ROI may be selected by a user via a user interface, such as user interface 224.

As indicated at block 1006, the at least one processor calculates a parametric value based, at least in part, on a corresponding one of the plurality of statistical distribution to generate a plurality of parametric values. The parametric value may include a signal-to-noise ratio, a Nakagami index, or a combination thereof in some examples.

The at least one processor generates from the plurality of parametric values a plurality of parametric maps corresponding to the individual image frames of the plurality of temporally spaced image frames as indicated at block 1008. In some examples, at least one processor may provide one or more of the parametric maps for display, for example, on display 238.

In some examples, the at least one processor uses the parametric maps to define a subsequence to generate an accumulation image (e.g., ToA image or a MVI image). The at least one processor selects a first frame of the plurality of temporally spaced image frames based, at least in part, on a first parametric map of the plurality of parametric maps comprising a first parametric value over a threshold value. In some examples, the threshold value is based, at least in part, on a tissue type included in the plurality of temporally spaced image frames. In other examples, the threshold value may be based, at least in part, by the user input provided via the user interface. In some examples, the at least one processor selects a second frame of the plurality of temporally spaced image frames based, at least in part, on a second parametric map of the plurality of parametric maps comprising a maximum parametric value of the plurality of parametric values The first frame and the second frame define, at least in part, a subsequence of the plurality of temporally spaced image frames, and the method further comprises combining the subsequence to generate an accumulation image.

In some examples, the at least one processor may use the parametric maps to segment one or more features from the image frames. For example, the at least one processor may determine a maximum parametric value of the plurality of parametric values corresponding to a first parametric map of the plurality of parametric maps and threshold the plurality of parametric values corresponding to remaining ones of the plurality of parametric maps to remove parametric values of the plurality of parametric values below the maximum parametric value. The at least one processor may segment at least one of the plurality of temporally spaced image frames corresponding to at least one of the remaining ones of the plurality of parametric maps. In some examples, segmenting may include assigning pixels of the at least one of the plurality of temporally spaced image frames to a feature, wherein the pixels correspond to parametric values equal to or greater than a threshold value.

In various examples where components, systems and/or methods are implemented using a programmable device, such as a computer-based system or programmable logic, it should be appreciated that the above-described systems and methods can be implemented using any of various known or later developed programming languages, such as "C", "C++", "FORTRAN", "Pascal", "VHDL" and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure it is noted that the various methods and devices described herein can be implemented in hardware, software, and/or firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention as defined by the appended claims. The functionality of one or more of the processors described herein may be incorporated into a fewer number or a single processing unit (e.g., a CPU) and may be implemented using application specific integrated circuits (ASICs) or general purpose processing circuits which are programmed responsive to executable instructions to perform the functions described herein.

Although the present system may have been described with particular reference to an ultrasound imaging system, it is also envisioned that the present system can be extended to other medical imaging systems where one or more images are obtained in a systematic manner. Accordingly, the present system may be used to obtain and/or record image information related to, but not limited to renal, testicular, breast, ovarian, uterine, thyroid, hepatic, lung, musculoskeletal, splenic, cardiac, arterial and vascular systems, as well as other imaging applications related to ultrasound-guided interventions. Further, the present system may also include one or more programs which may be used with conventional imaging systems so that they may provide features and advantages of the present system. Certain additional advantages and features of this disclosure may be apparent to those skilled in the art upon studying the disclosure, or may be experienced by persons employing the novel system and method of the present disclosure. Another advantage of the present systems and method may be that conventional medical image systems can be easily upgraded to incorporate the features and advantages of the present systems, devices, and methods.

Of course, it is to be appreciated that any one of the examples, examples or processes described herein may be combined with one or more other examples, examples and/or processes or be separated and/or performed amongst separate devices or device portions in accordance with the present systems, devices and methods.

## Claims

1. An apparatus (200) for processing ultrasound images, the apparatus comprising:
at least one processor (236, 300) configured to:
for individual ones of a plurality of temporally spaced image frames, calculate a plurality of parametric values based, at least in part, on statistical distributions of corresponding ones of a plurality of groups of pixels of a corresponding image frame of the plurality of temporally spaced image frames, wherein the plurality of groups of pixels are defined, at least in part, on a multi-pixel window translated across the image frame, wherein the plurality of temporally spaced image frames comprise contrast enhanced ultrasound images;
generate a plurality of parametric maps comprising the plurality of parametric values, wherein individual ones of the plurality of parametric maps correspond to the individual ones of the plurality of temporally spaced image frames;
select a first frame from the plurality of temporally spaced image frames based, at least in part, on determining a parametric map of the plurality of parametric maps comprising a parametric value equal to or above a threshold value;
select a last frame from the plurality of temporally spaced image frames based, at least in part, on determining a second parametric map of the plurality of parametric maps comprising a maximum parametric value; and
combine the first frame, the last frame, and any image frame of the plurality of temporally spaced image frames spaced between the first frame and the last frame to generate an accumulation image.

2. The apparatus (200) of claim 1, wherein the last frame corresponds to the second parametric map.

3. The apparatus (200) of claim 1, wherein the accumulation image comprises a time-of-arrival image.

4. The apparatus (200) of claim 3, wherein at least one of a temporal gradient or a coding range of the time-of-arrival image is based, at least in part, on the plurality of parametric maps.

5. The apparatus (200) of claim 1, wherein the first frame corresponds to the parametric map of the plurality of parametric maps comprising the parametric value equal to or above the threshold value.

6. The apparatus (200) of claim 1, wherein at least one of the plurality of parametric maps corresponds to an image frame of the plurality of temporally spaced image frames acquired prior to arrival of a contrast agent, and the at least one processor is further configured to determine a maximum value of the plurality of parametric values of the at least one of the plurality of parametric maps; and
threshold remaining ones of the plurality of parametric maps corresponding to image frames of the plurality of temporally spaced image frames acquired after the image frame of the plurality of temporally spaced image frames acquired prior to the arrival of the contrast agent.

7. The apparatus (200) of claim 6, wherein the at least one processor is further configured to segment a feature from at least one image frame of the image frames of the plurality of temporally spaced image frames acquired after the image frame of the plurality of temporally spaced image frames acquired prior to arrival of the contrast agent based, at least in part, on corresponding thresholded ones of the remaining ones of the plurality of parametric maps.

8. A processor-implemented method (1000) comprising:
translating (1002) a multi-pixel window across individual image frames of a plurality of temporally spaced image frames, wherein the plurality of temporally spaced image frames comprise contrast enhanced ultrasound images;
for each translation of the multi-pixel window:
determining (1004) a statistical distribution of pixels of the individual image frames included in the multi-pixel window to generate a plurality of statistical distributions;
calculating (1006) a parametric value based, at least in part, on a corresponding one of the plurality of statistical distribution to generate a plurality of parametric values;
generating (1008) from the plurality of parametric values a plurality of parametric maps corresponding to the individual image frames of the plurality of temporally spaced image frames;
selecting a first frame of the plurality of temporally spaced image frames based, at least in part, on a first parametric map of the plurality of parametric maps comprising a first parametric value over a threshold value; and
selecting a last frame of the plurality of temporally spaced image frames based, at least in part, on a second parametric map of the plurality of parametric maps comprising a maximum parametric value of the plurality of parametric values, combining the first frame, the last frame, and any image frame of the plurality of temporally spaced image frames spaced between the first frame and the last frame to generate an accumulation image.

9. The method (1000) of claim 8, wherein the parametric value comprises at least one of a signal-to-noise ratio, a Nakagami index, or a combination thereof.

10. The method (1000) of claim 8, wherein the multi-pixel window comprises a square.

11. The method (1000) of claim 8, wherein the threshold value is based, at least in part, on a tissue type included in the plurality of temporally spaced image frames.

12. The method (1000) of claim 8, further comprising:
determining a maximum parametric value of the plurality of parametric values corresponding to a first parametric map of the plurality of parametric maps; and
thresholding the plurality of parametric values corresponding to remaining ones of the plurality of parametric maps to remove parametric values of the plurality of parametric values below the maximum parametric value.

13. The method (1000) of claim 12, further comprising segmenting at least one of the plurality of temporally spaced image frames corresponding to at least one of the remaining ones of the plurality of parametric maps.

14. The method (1000) of claim 13, wherein segmenting comprises assigning pixels of the at least one of the plurality of temporally spaced image frames to a feature, wherein the pixels correspond to parametric values equal to or greater than a threshold value.

15. The method (1000) of claim 8, wherein the multi-pixel window is only translated across a region of interest in the individual image frames of the plurality of temporally spaced image frames, wherein the region of interest is smaller than the individual image frames.

## Patentansprüche

1. Einrichtung (200) zum Verarbeiten von Ultraschallbildern, wobei die Einrichtung Folgendes umfasst:
mindestens einen Prozessor (236, 300), konfiguriert, um:
für einzelne von einer Vielzahl von zeitlich beabstandeten Bildrahmen eine Vielzahl von parametrischen Werten zu berechnen, die, mindestens teilweise, auf statistischen Verteilungen entsprechender von einer Vielzahl von Gruppen von Pixeln eines entsprechenden Bildrahmens von der Vielzahl von zeitlich beabstandeten Bildrahmen basieren, wobei die Vielzahl von Pixelgruppen, mindestens teilweise, durch ein über den Bildrahmen verschobenes Mehrpixelfenster definiert sind, wobei die Vielzahl von zeitlich beabstandeten Bildrahmen kontrastverstärkte Ultraschallbilder umfasst;
eine Vielzahl von parametrischen Karten zu erzeugen, welche die Vielzahl von parametrischen Werten umfassen, wobei einzelne von der Vielzahl von parametrischen Karten den einzelnen von der Vielzahl von zeitlich beabstandeten Bildrahmen entsprechen;
einen ersten Rahmen aus der Vielzahl von zeitlich beabstandeten Bildrahmen auszuwählen, basierend, mindestens teilweise, auf dem Bestimmen einer parametrischen Karte von der Vielzahl von parametrischen Karten, die einen parametrischen Wert umfasst, der so groß wie oder größer als ein Schwellenwert ist;
einen letzten Rahmen aus der Vielzahl von zeitlich beabstandeten Bildrahmen auszuwählen, basierend, mindestens teilweise, auf dem Bestimmen einer zweiten parametrischen Karte von der Vielzahl von parametrischen Karten, die einen maximalen parametrischen Wert umfasst; und
den ersten Rahmen, den letzten Rahmen und einen beliebigen Rahmen von der Vielzahl von zeitlich beabstandeten Bildrahmen, die zwischen dem ersten Rahmen und dem letzten Rahmen beabstandet sind, zu kombinieren, um ein Akkumulationsbild zu erzeugen.

2. Einrichtung (200) nach Anspruch 1, wobei der letzte Rahmen der zweiten parametrischen Karte entspricht.

3. Einrichtung (200) nach Anspruch 1, wobei das Akkumulationsbild ein Ankunftszeitbild umfasst.

4. Einrichtung (200) nach Anspruch 3, wobei mindestens eines von einem zeitlichen Gradienten oder einem Codierungsbereich des Ankunftszeitbildes, mindestens teilweise, auf der Vielzahl von parametrischen Karten basiert.

5. Einrichtung (200) nach Anspruch 1, wobei der erste Rahmen der parametrischen Karte von der Vielzahl von parametrischen Karten entspricht, die den parametrischen Wert umfasst, der so groß wie oder größer als der Schwellenwert ist.

6. Einrichtung (200) nach Anspruch 1, wobei mindestens eine von der Vielzahl von parametrischen Karten einem Bildrahmen von der Vielzahl von zeitlich beabstandeten Bildrahmen entspricht, die vor dem Eintreffen eines Kontrastmittels erfasst wurden, und der mindestens eine Prozessor weiter so konfiguriert ist, dass er einen Maximalwert von der Vielzahl von parametrischen Werten der mindestens einen von der Vielzahl von parametrischen Karten bestimmt; und
Schwellenwerte von verbleibenden von der Vielzahl von parametrischen Karten zu bestimmen, die Bildrahmen von der Vielzahl von zeitlich beabstandeten Bildrahmen entsprechen, die nach dem Bildrahmen von der Vielzahl von zeitlich beabstandeten Bildrahmen erfasst wurden, die vor dem Eintreffen des Kontrastmittels erfasst wurden.

7. Einrichtung (200) nach Anspruch 6, wobei der mindestens eine Prozessor weiter so konfiguriert ist, dass er ein Merkmal aus mindestens einem Bildrahmen von den Bildrahmen von der Vielzahl von zeitlich beabstandeten Bildrahmen, die nach dem Bildrahmen von der Vielzahl von zeitlich beabstandeten Bildrahmen aufgenommen wurden, die vor dem Eintreffen des Kontrastmittels aufgenommen wurden, basierend, mindestens teilweise, auf entsprechenden schwellenwertbasierten der verbleibenden von der Vielzahl von parametrischen Karten segmentiert.

8. Prozessorimplementiertes Verfahren (1000), umfassend:
Verschieben (1002) eines Mehrpixelfensters über einzelne Bildrahmen einer Vielzahl von zeitlich beabstandeten Bildrahmen, wobei die Vielzahl von zeitlich beabstandeten Bildrahmen kontrastverstärkte Ultraschallbilder umfasst;
für jede Verschiebung des Mehrpixelfensters:
Bestimmen (1004) einer statistischen Verteilung von Pixeln der einzelnen Bildrahmen, die im Mehrpixelfenster eingeschlossen sind, um eine Vielzahl von statistischen Verteilungen zu erzeugen;
Berechnen (1006) eines parametrischen Werts, der, mindestens teilweise, auf einer entsprechenden von der Vielzahl von statistischen Verteilungen basiert, um eine Vielzahl von parametrischen Werten zu erzeugen;
Erzeugen (1008) aus der Vielzahl von parametrischen Werten einer Vielzahl von parametrischen Karten, die den einzelnen Bildrahmen von der Vielzahl von zeitlich beabstandeten Bildrahmen entsprechen;
Auswählen eines ersten Rahmens aus der Vielzahl von zeitlich beabstandeten Bildrahmen, basierend, mindestens teilweise, auf einer ersten parametrischen Karte von der Vielzahl von parametrischen Karten, die einen ersten parametrischen Wert über einem Schwellenwert umfasst; und
Auswählen eines letzten Rahmens aus der Vielzahl von zeitlich beabstandeten Bildrahmen, basierend, mindestens teilweise, auf einer zweiten parametrischen Karte von der Vielzahl von parametrischen Karten, die einen maximalen parametrischen Wert von der Vielzahl von parametrischen Werten umfasst,
Kombinieren des ersten Rahmens, des letzten Rahmens und eines beliebigen Rahmens von der Vielzahl von zeitlich beabstandeten Bildrahmen, die zwischen dem ersten Rahmen und dem letzten Rahmen beabstandet sind, um ein Akkumulationsbild zu erzeugen.

9. Verfahren (1000) nach Anspruch 8, wobei der parametrische Wert mindestens eines von einem Signal-Rausch-Verhältnis, einem Nakagami-Index oder einer Kombination davon umfasst.

10. Verfahren (1000) nach Anspruch 8, wobei das Mehrpixelfenster ein Quadrat umfasst.

11. Verfahren (1000) nach Anspruch 8, wobei der Schwellenwert, mindestens teilweise, auf einem Gewebetyp basiert, der in der Vielzahl von zeitlich beabstandeten Bildrahmen eingeschlossen ist.

12. Verfahren (1000) nach Anspruch 8, das weiter Folgendes umfasst:
Bestimmen eines maximalen parametrischen Werts von der Vielzahl von parametrischen Werten, die einer ersten Parameterkarte von der Vielzahl der Parameterkarten entsprechen; und
Schwellenwertbildung der Vielzahl von parametrischen Werten, die verbleibenden von der Vielzahl von parametrischen Karten entsprechen, um parametrische Werte von der Vielzahl von parametrischen Werten unterhalb des maximalen parametrischen Wertes zu entfernen.

13. Verfahren (1000) nach Anspruch 12, weiter umfassend das Segmentieren mindestens eines von der Vielzahl von zeitlich beabstandeten Bildrahmen, die mindestens einem der verbleibenden von der Vielzahl von parametrischen Karten entsprechen.

14. Verfahren (1000) nach Anspruch 13, wobei das Segmentieren das Zuordnen von Pixeln des mindestens einen von der Vielzahl von zeitlich beabstandeten Bildrahmen zu einem Merkmal umfasst, wobei die Pixel parametrischen Werten entsprechen, die so groß wie oder größer als ein Schwellenwert sind.

15. Verfahren (1000) nach Anspruch 8, wobei das Mehrpixelfenster nur über einen Bereich von Interesse in den einzelnen Bildrahmen der Vielzahl von zeitlich beabstandeten Bildrahmen verschoben wird, wobei der Bereich von Interesse kleiner ist als die einzelnen Bildrahmen.

## Revendications

1. Appareil (200) pour le traitement d'images ultrasonores, l'appareil comprenant :
au moins un processeur (236, 300) configuré pour :
pour chaque image d'une pluralité de trames d'images espacées dans le temps, calculer une pluralité de valeurs paramétriques basées, au moins en partie, sur des distributions statistiques de groupes correspondants d'une pluralité de groupes de pixels d'une trame d'image correspondante de la pluralité de trames d'images espacées dans le temps, dans lequel la pluralité de groupes de pixels sont définis, au moins en partie, sur une fenêtre multipixels translatée à travers la trame d'image, dans lequel la pluralité de trames d'images espacées dans le temps comprennent des images ultrasonores à contraste amélioré ;
générer une pluralité de cartes paramétriques comprenant la pluralité de valeurs paramétriques, dans lequel des cartes individuelles de la pluralité de cartes paramétriques correspondent à des trames individuelles de la pluralité de trames d'image de la pluralité espacées dans le temps ;
sélectionner une première trame parmi la pluralité de trames d'images espacées dans le temps en se basant, au moins en partie, sur la détermination d'une carte paramétrique parmi la pluralité de cartes paramétriques comprenant une valeur paramétrique égale ou supérieure à une valeur seuil ;
sélectionner une dernière trame parmi la pluralité de trames d'images espacées dans le temps, en se basant, au moins en partie, sur la détermination d'une seconde carte paramétrique parmi la pluralité de cartes paramétriques comprenant une valeur paramétrique maximale ; et
combiner la première trame, la dernière trame et n'importe quelle trame d'image parmi la pluralité de trames d'images espacées dans le temps entre la première et la dernière trame pour générer une image d'accumulation.

2. Appareil (200) selon la revendication 1, dans lequel la dernière trame correspond à la seconde carte paramétrique.

3. Appareil (200) selon la revendication 1, dans lequel l'image d'accumulation comprend une image de temps d'arrivée.

4. Appareil (200) selon la revendication 3, dans lequel au moins un gradient temporel ou une plage de codage de l'image du temps d'arrivée est basé(e), au moins en partie, sur la pluralité de cartes paramétriques.

5. Appareil (200) selon la revendication 1, dans lequel la première trame correspond à la carte paramétrique de la pluralité de cartes paramétriques comprenant la valeur paramétrique égale ou supérieure à la valeur seuil.

6. Appareil (200) selon la revendication 1, dans lequel au moins une de la pluralité de cartes paramétriques correspond à une trame d'image parmi la pluralité de trames d'images espacées dans le temps acquises avant l'arrivée d'un agent de contraste, et l'au moins un processeur est en outre configuré pour déterminer une valeur maximale parmi la pluralité de valeurs paramétriques de l'au moins une de la pluralité de cartes paramétriques ; et
établir un seuil pour les valeurs paramétriques restantes parmi la pluralité de cartes paramétriques correspondant aux trames d'images de la pluralité de trames d'images espacées dans le temps acquises après la trame d'image de la pluralité de trames d'images espacées dans le temps acquise avant l'arrivée de l'agent de contraste.

7. Appareil (200) selon la revendication 6, dans lequel l'au moins un processeur est en outre configuré pour segmenter une caractéristique d'au moins une trame d'image parmi les trames d'images de la pluralité de trames d'images espacées dans le temps, acquises après la trame d'image de la pluralité de trames d'images espacées dans le temps, acquise avant l'arrivée de l'agent de contraste, en se basant, au moins en partie, sur les cartes restantes correspondantes de la pluralité de cartes paramétriques pour lesquelles un seuil a été établi.

8. Procédé mis en œuvre par un processeur (1000) comprenant :
la translation (1002) d'une fenêtre multipixels à travers des trames d'images individuelles d'une pluralité de trames d'images espacées dans le temps, dans lequel la pluralité de trames d'images espacées dans le temps comprennent des images ultrasonores à contraste amélioré ;
pour chaque translation de la fenêtre multipixels :
la détermination (1004) d'une distribution statistique des pixels des trames d'image individuelles incluses dans la fenêtre multipixels pour générer une pluralité de distributions statistiques ;
le calcul (1006) d'une valeur paramétrique basée, au moins en partie, sur une valeur correspondante de la pluralité de distributions statistiques pour générer une pluralité de valeurs paramétriques ;
la génération (1008) à partir de la pluralité de valeurs paramétriques d'une pluralité de cartes paramétriques correspondant aux trames d'images individuelles de la pluralité de trames d'images espacées dans le temps ;
la sélection d'une première trame parmi la pluralité de trames d'images espacées dans le temps, basée au moins en partie sur une première carte paramétrique parmi la pluralité de cartes paramétriques comprenant une première valeur paramétrique supérieure à une valeur seuil ; et
la sélection d'une dernière trame parmi la pluralité de trames d'images espacées dans le temps, basée au moins en partie sur une seconde carte paramétrique parmi la pluralité de cartes paramétriques comprenant une valeur paramétrique maximale parmi la pluralité de valeurs paramétriques,
la combinaison de la première trame, la dernière trame et n'importe quelle trame parmi la pluralité de trames d'images espacées dans le temps entre la première et la dernière trame pour générer une image d'accumulation

9. Procédé (1000) selon la revendication 8, dans lequel la valeur paramétrique comprend au moins un rapport signal/bruit, un indice de Nakagami ou une combinaison de ceux-ci.

10. Procédé (1000) selon la revendication 8, dans lequel la fenêtre multipixels comprend un carré.

11. Procédé (1000) selon la revendication 8, dans lequel la valeur seuil est basée, au moins en partie, sur un type de tissu inclus dans la pluralité de trames d'images espacées dans le temps.

12. Procédé (1000) selon la revendication 8, comprenant en outre :
la détermination d'une valeur paramétrique maximale parmi la pluralité de valeurs paramétriques correspondant à une première carte paramétrique parmi la pluralité de cartes paramétriques ; et
l'établissement d'un seuil de la pluralité des valeurs paramétriques correspondant aux cartes restantes de la pluralité des cartes paramétriques afin de supprimer des valeurs paramétriques de la pluralité de valeurs paramétriques inférieures à la valeur paramétrique maximale.

13. Procédé (1000) selon la revendication 12, comprenant en outre la segmentation d'au moins une des pluralités de trames d'images espacées dans le temps correspondant à au moins une des cartes restantes de la pluralité de cartes paramétriques.

14. Procédé (1000) selon la revendication 13, dans lequel la segmentation comprend l'attribution de pixels de l'au moins une de la pluralité de trames d'images espacées dans le temps à une caractéristique, dans lequel les pixels correspondent à des valeurs paramétriques égales ou supérieures à une valeur seuil.

15. Procédé (1000) selon la revendication 8, dans lequel la fenêtre multipixels est uniquement translatée à travers une région d'intérêt dans les trames d'images individuelles de la pluralité de trames d'images espacées dans le temps, dans lequel la région d'intérêt est plus petite que les trames d'images individuelles.
